# FASCICULE DE BREVET EUROPEEN

(11) **EP 3 943 049 B1**
(45) Date de publication et mention de la délivrance du brevet: **24.05.2023**
(21) Numéro de dépôt: 20315352.3
(22) Date de dépôt: 21.07.2020
(51) Int. Cl.: A61F 2/44, A61F 2/30

(54) **CAGE INTEROSSEUSE**
INTEROSSÄRES GERÜST
INTEROSSEOUS CAGE

(43) Date de publication de la demande: 26.01.2022
(73) Titulaire: Razian, Hassan, 94240 L'Haÿ-les-Roses (FR)
(72) Inventeur: Razian, Sam, 94240 L'Haÿ-les-Roses (FR)
(74) Mandataire: Flavenot, Bernard

(56) Documents cités:
- WO-A1-2009/070721
- WO-A1-2018/081140
- FR-A1- 3 092 246
- US-A1- 2003 149 484
- US-A1- 2011 035 007
- US-B1- 8 545 562

## Description

### Domaine technique

La présente invention concerne les cages interosseuses, à savoir les cages aptes à être implantées entre deux portions d'os en vue de favoriser leur solidarisation de façon ferme par ostéosynthèse, qui trouvent des applications particulièrement avantageuses, mais non exclusivement, comme cages intervertébrales.

On connaît déjà de nombreuses cages interosseuses de ce type. Elles sont essentiellement composées d'un corps solide, par exemple en métal ou PEEK, comportant une cavité traversante dans laquelle est placé un greffon osseux ou substitut osseux qui permet de favoriser la croissance osseuse et donc la liaison entre les deux portions d'os par ostéosynthèse.

De telles cages sont connues notamment par le US 8545562, le WO2018081140, le US 2011035007, le US 2003149484 et le WO2009070721.

On connaît aussi une cage comme celle qui est décrite et illustrée dans le US6770096, comportant en outre une lame et une dent qui est dans le plan de la lame. Cette réalisation permet d'éviter que la lame se retire de la portion osseuse dans laquelle elle est implantée et que cette portion osseuse ne reste plus au contact du corps solide.

Dans ce but, pour que la dent puisse pénétrer dans la partie osseuse, elle doit être sensiblement de la même épaisseur que la lame, c'est-à-dire d'une épaisseur très faible. Il en résulte qu'elle se désolidarise relativement facilement de la lame, essentiellement pas le fait qu'elle casse sous la moindre traction. En outre, l'implantation de la lame et de la dent dans la partie osseuse est relativement délicate et contribue à une destruction de cette partie osseuse qui est relativement longue à se reconstituer. C'est pour cela que ce type de cage n'a jamais été réellement mis en oeuvre.

Aussi, la présente invention a-t-elle pour but de réaliser une cage interosseuse qui pallie l'inconvénient des cages de l'art antérieur exposé ci-dessus.

### Définition de l'invention

Plus précisément, la présente invention a pour objet une cage interosseuse apte à être positionnée entre au moins deux portions d'os en vue de leur réunion par ostéosynthèse, telle que définie dans au moins l'une les revendications annexées.

### Brève Description des dessins

D'autres caractéristiques et avantages de la présente invention apparaîtront au cours de la description suivante donnée en regard des dessins annexés à titre illustratif, mais nullement limitatif, dans lesquels :
- La figure 1 représente une vue en coupe longitudinale schématique d'un mode de réalisation de l'un des éléments constitutifs de la cage interosseuse selon l'invention, et
- La figure 2 représente une vue en perspective cavalière d'une cage selon l'invention faisant application du mode de réalisation de l'élément illustré sur la figure 1, dans une application comme cage intervertébrale.

Il est précisé que, dans la présente description, si l'adverbe "sensiblement" est associé à un qualificatif d'un moyen donné, ce qualificatif doit être compris au sens strict ou approché.

### Description de modes de réalisations préférés de l'objet de l'invention.

La présente invention est relative à une cage interosseuse apte à être positionnée contre au moins une portion d'os O1 en vue d'une ostéosynthèse entre cette portion d'os et une autre portion d'os (non représentée), qui trouve une application particulièrement avantageuse, mais non exclusivement, comme cage intervertébrale. La cage comporte, comme dans l'art antérieur, au moins un corps 10 délimité par deux faces opposées 11, 12, et au moins une lame 20 définissant, dans sa forme générale, un plan de coupe 21. La lame présente de ce fait, comme pour un couteau, un bord en fil de coupe 25 pour sa pénétration dans la portion d'os O1.

La cage comporte en outre des moyens 30 pour monter la lame 20 en rotation par rapport au corps 10, la rotation étant apte à s'effectuer autour d'un axe de rotation 31 sensiblement perpendiculaire au plan de coupe 21 de façon que, lors de la rotation de la lame, au moins une partie du bord en fil de coupe 25 pénètre dans la portion d'os O1.

La cage comporte en outre au moins une contre-lame 40 ayant, elle aussi, un bord en fil de coupe pour sa pénétration dans un milieu osseux, solidarisée en saillie sur l'une 22 des deux faces de la lame 20.

Selon la caractéristique essentielle de l'invention, la au moins une contre-lame 40 est obtenue par une déformation de la lame 20 sous la forme d'une ondulation 45 réalisée dans un plan sensiblement perpendiculaire au plan de coupe 21 et contenant l'axe de rotation 31.

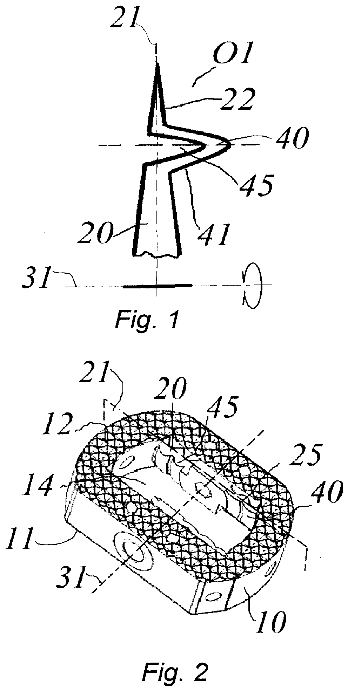

De façon avantageuse, la cage comporte cependant une pluralité de contre-lames 40, figure 2, les contre-lames de cette pluralité de contre-lames étant espacées les unes des autres sur la face 22 (figure 1) de la lame 20.

Selon une autre caractéristique préférentielle de l'invention, la longueur de chacune des contre-lames 40 de la pluralité de ces contre-lames, longueur prise à partir du plan de coupe 31 de la lame 20, est sensiblement inversement proportionnelle à la distance séparant chaque contre-lame de l'axe de rotation 31.

De façon également très avantageuse, les bords en fil de coupe des contre-lames 40 de la pluralité sont sensiblement coplanaires et, encore plus préférentiellement, les bords en fil de coupe des contre-lames 40 de la pluralité et le bord en fil de coupe 25 de la lame 20 sont coplanaires.

Les caractéristiques décrites ci-dessus permettent de moduler les efforts de pénétration de la lame 20 dans la portion d'os O1 au fur et à mesure de sa rotation et de sa pénétration dans l'os.

Il est précisé qu'il peut être prévu des contre-lames en saillie sur les deux faces opposées de la lame.

Par ailleurs, il est préférable que le corps 10 comporte une cavité traversante 14, figure 2, débouchant sur ses deux faces opposées 11, 12, dans le but exposé au préambule. Dans ce cas, les contre-lames de la pluralité sont préférentiellement en saillie sur une même face 22 de la lame et cette dernière est montée en rotation dans la cavité 14 de façon que sa face, ne portant pas de contre-lame 40, soit au moins partiellement sensiblement au contact de la paroi de la cavité 14, comme visible sur la figure 2.

Cette caractéristique permet d'aménager un maximum d'espace libre dans la cavité traversante 14 pour, de façon connue en elle-même, pouvoir y placer par exemple des substituts osseux ou analogues.

De même, cette cage peut avantageusement comporter par exemple deux lames 20 respectivement situées dans deux plans parallèles non confondus, avec au moins une contre-lame 40 associée à chaque lame (figure 2).

Il est cependant précisé que, selon un autre mode de réalisation non illustré, les moyens pour monter l'ensemble lame 20/contre-lame 40 en rotation par rapport au corps 10 peuvent être agencés de façon que l'axe de rotation 31 fasse, avec le plan de coupe 21, un angle de valeur non nulle différente de 90°.

Selon un autre mode de réalisation possible, la cage peut alors comporter deux lames dont les plans de coupe ne sont pas parallèles (par exemple faisant respectivement 60° et 120° avec l'axe de rotation).

Cette structure permet aux deux lames de réaliser entre elles un effet de coin qui contribue, en combinaison avec la forme des contre-lames 40 en saillie, à optimiser la solidarisation de la cage avec la portion d'os à laquelle cette cage est associée et à éviter que la cage mouve par rapport à cette portion d'os.

La réalisation des moyens pour monter l'ensemble lame 20/contre-lame 40 en rotation par rapport au corps 10 ne pose pas de problème à un homme du métier. Elle se déduit facilement de l'art antérieur comme celui qui est indiqué au préambule de la présente description, la figure 2 indiquant une solution à cet homme du métier. A la lecture de la description ci-dessus, il apparaît clairement que la cage interosseuse selon l'invention permet de maintenir la distance entre deux portions d'os, à l'aide d'un ancrage sécurisé de la lame 20 dans le milieu osseux induisant en plus, lors de l'introduction d'au moins une lame avec son ondulation (ou ses ondulations), une destruction osseuse moins importante qu'avec les cages de l'art antérieur comme celles mentionnées au préambule de la présente description.

La cage interosseuse selon l'invention ne présente pas l'inconvénient évoqué dans ce même préambule.

## Revendications

1. Cage interosseuse apte à être positionnée contre au moins une portion d'os (O1) en vue d'une ostéosynthèse entre cette portion d'os et une autre portion d'os, ladite cage comportant au moins :
• un corps (10) délimité par deux faces opposées (11, 12),
• au moins une lame (20) sensiblement plane définissant un plan de coupe (21), ladite lame présentant au moins un bord en fil de coupe (25) pour sa pénétration dans ladite portion d'os (O1),
• des moyens pour monter ladite lame (20) en rotation par rapport audit corps (10), ladite rotation étant apte à s'effectuer autour d'un axe de rotation (31) de façon qu'au moins une partie du bord en fil de coupe soit apte à pénétrer dans ladite portion d'os (O1), et
• au moins une contre-lame (40) ayant aussi un bord en fil de coupe, ladite contre-lame étant solidarisée en saillie sur l'une (22) des deux faces de ladite lame (20),
**caractérisée par le fait que** la au moins une contre-lame (40) est obtenue par une déformation de ladite lame (20) sous la forme d'une ondulation (45) réalisée dans un plan sensiblement perpendiculaire au plan de coupe (21) et contenant ledit axe de rotation (31).

2. Cage selon la revendication 1, **caractérisée par le fait qu'**elle comporte une pluralité de contre-lames (40), les contre-lames de ladite pluralité de contre-lames étant espacées les unes des autres sur ladite une face (22) de ladite lame (20).

3. Cage selon la revendication 2, **caractérisée par le fait que** la longueur de chacune des contre-lames (40) de ladite pluralité, longueur prise à partir du plan de coupe (31) de ladite lame (20), est sensiblement inversement proportionnelle à la distance séparant chaque contre-lame du dit axe de rotation (31).

4. Cage selon l'une des revendications 2 et 3, **caractérisée par le fait que** les bords en fil de coupe des contre-lames (40) de ladite pluralité sont sensiblement coplanaires.

5. Cage selon la revendication 4, **caractérisée par le fait que** les bords en fil de coupe des contre-lames (40) de ladite pluralité et le bord en fil de coupe (25) de la lame (20) sont coplanaires.

6. Cage selon l'une des revendications précédentes, **caractérisée par le fait que**, ledit corps (10) comportant une cavité traversante (14) débouchant sur ses deux faces opposées (11, 12) et l'une des deux faces de la lame (20) ne portant pas de contre-lame (40), ladite lame est montée en rotation dans ladite cavité de façon que sa face ne portant pas de contre-lame (40) soit au moins partiellement sensiblement au contact de la paroi de ladite cavité (14).

7. Cage selon l'une des revendications précédentes, **caractérisée par le fait qu'**elle comporte au moins deux lames (20) respectivement situées dans deux plans parallèles et non confondus, et au moins une contre-lame (40) associée à chaque lame.

8. Cage selon l'une des revendications précédentes, **caractérisée par le fait que** les moyens pour monter ladite lame (20) en rotation par rapport audit corps (10) sont agencés de façon que le plan de coupe (21) de la lame fasse, avec ledit axe de rotation (31), un angle de valeur non nulle.

## Patentansprüche

1. Interossärer Käfig, der positionierbar ist an mindestens einem Knochenabschnitt (O1) im Hinblick auf eine Osteosynthese zwischen diesem Knochenabschnitt und einem anderen Knochenabschnitt, wobei der Käfig mindestens umfasst:
einen Körper (10), der von zwei gegenüberliegende Seiten (11,12) begrenzt ist,
- mindestens eine im Wesentlichen ebene Klinge (20), die eine Schnittebene (21) definiert, wobei die Klinge mindestens eine als Schneiddraht wirkende Kante (25) aufweist, um in den Knochenabschnitt (O1) einzudringen,
' - Mittel zum Montieren der Klinge (20) für eine Drehung in Bezug auf den Körper (10), wobei die Drehung um eine Drehachse (31) erfolgen kann, derart, dass mindestens ein Teil der als Schneiddraht wirkenden Kante in diesen Knochenabschnitt (O1) eindringen kann, und
- , mindestens eine Gegenklinge (40), die ebenfalls eine als Schneiddraht - wirkende Kante hat, wobei die Gegenklinge vorstehend fest mit einer (22) der beiden Seiten der Klinge (20) verbunden ist,
**dadurch gekennzeichnet, dass** die mindestens eine Gegenklinge (40) durch eine Verformung der Klinge (20) in Form einer Wellung (45) erhalten wird, die in einer Ebene ausgeführt ist, die im Wesentlichen senkrecht zur Schneidebene (21) verläuft und die Drehachse (31) enthält.

2. Käfig nach Anspruch 1, **dadurch gekennzeichnet, dass** er eine Mehrzahl von Gegenklingen (40) aufweist, wobei die Gegenklingen der Mehrzahl von Gegenklingen auf der einen Seite (22) der Klinge (20) voneinander beabstandet sind.

3. Käfig nach Anspruch 2, **dadurch gekennzeichnet, dass** die Länge jeder der Gegenklingen (40) der Mehrzahl, die ausgehend von der Schnittebene (31) der Klinge (20) gemessen wird, zu dem Abstand, der jede Gegenklinge von der Drehachse (31) trennt, im Wesentlichen umgekehrt proportional ist.

4. Käfig nach einem der Ansprüche 2 und 3, **dadurch gekennzeichnet, dass** die als Schneiddraht wirkenden Kanten der Gegenklingen (40) der Mehrzahl im Wesentlichen koplanar sind.

5. Käfig nach Anspruch 4, **dadurch gekennzeichnet, dass** die als Schneiddraht wirkenden Kanten der Gegenklingen (40) der Mehrzahl und die als Schneiddraht wirkende Kante (25) der Klinge (20) koplanar sind.

6. Käfig nach einem der vorhergehenden Ansprüche, **dadurch gekennzeichnet, dass** der Körper (10) einen durchgehenden Hohlraum (14) aufweist, der an seinen beiden gegenüberliegenden Seiten (11,12) mündet, und eine der beiden Seiten der Klinge (20) keine Gegenklinge (40) trägt, wobei die Klinge in dem Hohlraum drehbar montiert ist, so dass ihre keine Gegenklinge (40) tragende Seite zumindest teilweise im Wesentlichen in Kontakt mit der Wand des Hohlraums (14) steht.

7. Käfig nach einem der vorhergehenden Ansprüche, **dadurch gekennzeichnet, dass** er mindestens zwei Klingen (20), die jeweils in zwei parallelen und nicht zusammenfallenden Ebenen liegen, und mindestens eine Gegenklinge (40), die jeder Klinge zugeordnet ist, umfasst.

8. Käfig nach einem der vorhergehenden Ansprüche, **dadurch gekennzeichnet, dass** die Mittel für drehbare Anbringung der Klinge (20) in Bezug auf den Körper (10) so angeordnet sind, dass die Schneidebene (21) der Klinge mit der Drehachse (31) einen Winkel mit einem Wert ungleich Null bildet.

## Claims

1. An interosseous cage able of being positioned against at least one bone portion (O1) for osteosynthesis between this bone portion and another bone portion, said cage comprising at least:
a body (10) delimited by two opposite faces (11, 12),
• at least one substantially flat blade (20) defining a cutting plane (21), said blade having at least one cutting wire edge (25) for its penetration into said bone portion (O1),
• means for rotatably mounting said blade (20) with respect to said body (10), said rotation being able to take place about a rotation axis (31) so that at least a portion of the cutting wire edge is able to penetrate into said bone portion (O1), and
• at least one counter-blade (40) also having a cutting wire edge, said counter-blade being secured in projecting manner on one (22) of the two faces of said blade (20),
**characterised in that** the at least one counter-blade (40) is obtained by a deformation of said blade (20) in the form of a corrugation (45) produced in a plane substantially perpendicular to the cutting plane (21) and containing said rotation axis (31).

2. The cage according to claim 1, **characterised in that** it comprises a plurality of counter-blades (40), the counter-blades of said plurality of counter-blades being spaced apart from one another on said one face (22) of said blade (20).

3. The cage according to claim 2, **characterised in that** the length of each of the counter-blades (40) of said plurality, taken from the cutting plane (31) of said blade (20), is substantially inversely proportional to the distance separating each counter-blade from said rotation axis (31).

4. The cage according to any one of claims 2 and 3, **characterised in that** the cutting wire edges of the counter-blades (40) of the said plurality are substantially coplanar.

5. The cage according to claim 4, **characterised in that** the cutting wire edges of the counter-blades (40) of said plurality and the cutting wire edge (25) of the blade (20) are coplanar.

6. The cage according to any one of the preceding claims, **characterised in that**, said body (10) comprising a through cavity (14) opening onto its two opposite faces (11, 12) and one of the two faces of the blade (20) not carrying a counter-blade (40), said blade is rotatably mounted in said cavity so that its face not carrying a counter-blade (40) is at least partially substantially in contact with the wall of said cavity (14).

7. The cage according to any one of the preceding claims, **characterised in that** it comprises at least two blades (20) respectively arranged in two parallel and not coincident planes, and at least one counter-blade (40) associated with each blade.

8. The cage according to any one of the preceding claims, **characterised in that** the means for rotatably mounting said blade (20) with respect to said body (10) are arranged in such a way that the cutting plane (21) of the blade forms, with said rotation axis (31), an angle of non-zero value.
